# FASCICULE DE BREVET EUROPEEN

(11) **EP 2 649 507 B1**
(45) Date de publication et mention de la délivrance du brevet: **30.01.2019**
(21) Numéro de dépôt: 11794476.9
(22) Date de dépôt: 09.12.2011
(51) Int. Cl.: G06F 3/044, B01L 3/00, A61B 5/145

(54) **DISPOSITIF MICRO-FLUIDIQUE POUR L'ANALYSE D'UN ÉCHANTILLON DE FLUIDE**
MIKROFLUIDISCHE VORRICHTUNG ZUR ANALYSE EINER FLÜSSIGKEITSPROBE
MICRO-FLUIDIC DEVICE FOR THE ANALYSIS OF A FLUID SAMPLE

(30) Priorité: 09.12.2010 CH 20542010
(43) Date de publication de la demande: 16.10.2013
(73) Titulaire: Qloudlab SA, 1095 Lutry (CH)
(72) Inventeur: QUEVAL, Arthur, 1095 Lutry (CH)
(74) Mandataire: Saam, Christophe
(86) Numéro de dépôt international: PCT/EP2011/072302
(87) Numéro de publication internationale: WO 2012/076683

(56) Documents cités:
- EP-A2- 2 237 137
- WO-A1-03/004160
- WO-A1-2005/001680
- US-A1- 2005 070 837
- US-A1- 2009 113 295
- US-A1- 2010 249 965
- US-A1- 2010 279 418

## Description

### Domaine technique

La présente invention concerne un dispositif micro-fluidique pour l'analyse d'un échantillon de fluide, et un système d'analyse d'échantillon de fluide comportant un tel dispositif. La présente invention concerne aussi un dispositif programmable avec un écran tactile capacitif contenant un processeur exécutant un logiciel pour analyser cet échantillon et afficher les résultats de cette analyse.

### Etat de la technique

Les puces micro-fluidiques sont largement utilisées dans le domaine des diagnostics pour la détection de pathologies diverses. Elles permettent d'effectuer une ou plusieurs analyses de façon rapide et précise sur une seule même puce en utilisant un très faible volume d'échantillon à analyser. Ces puces, connues aussi sous le nom de « biochip » ou « lab-on-a-chip » sont en général à usage unique et nécessitent d'une interface avec un ordinateur ou d'autres dispositifs afin d'afficher les résultats de l'analyse.

Afin de détecter l'échantillon, différentes méthodes sont utilisées, parmi lesquelles les méthodes de détection optiques, magnétiques, électromagnétiques, piézoélectriques, résistive et capacitives, ampérométrique et coulométrique.

Le signal de détection est ensuite converti en signal électrique pour subir des traitements (filtrage, échantillonnage, etc.) et permettre l'analyse des caractéristiques de l'échantillon de fluide, par exemple sa composition, température, vitesse du fluide, viscosité, conductivité, pH, etc.

Quelque soit la méthode de détection utilisée, chaque puce micro-fluidique et son interface sont généralement développées pour une application spécifique, ce qui diminue la modularité et la flexibilité de la puce.

L'intégration de l'électronique d'analyse dans une puce micro-fluidique connue renchérit son prix. Cette puce est en plus à usage unique, puisqu'il est difficile de la stériliser, vu les petites dimensions des ses micro-canaux pour l'échantillon de fluide.

Des techniques ont été développées pour augmenter la modularité de dispositifs micro-fluidiques en se servant de technologies existantes. Le document US20060166357 décrit un support de micro-fluidique dont les éléments telles que valves, pompes, etc. devant exercer une pression sur les micro-canaux sont activés par un élément externe tel qu'un écran tactile interactif par lequel la pression sur les canaux peut être exercé. En particulier, le support fonctionne avec un dispositif pour l'écriture Braille, qui peut être contrôlé par ordinateur à travers un simple éditeur de texte. Cependant le dispositif tactile sert seulement à l'entrée de commandes.

WO2008117223 décrit un biochip intégrant des actuateurs et/ou des capteurs disposés de façons matricielles pour interagir avec le dispositif micro-fluidique. Chaque actuateurs/capteurs peut être activé individuellement grâce à une matrice active de transistors fabriqués par technologie de films minces. Cependant, l'interprétation et l'analyse des résultats sont effectuées sur un dispositif externe. De plus, l'électronique doit être directement intégrée sur la puce augmentant de ce fait les coûts de fabrication du biochip.

US2010279418 concerne un dispositif de mesure du glucose ayant une porte destinée à recevoir une bandelette de test et une connexion pour se connecter à un dispositif portable tel qu'un IPhone. Le dispositif de
mesure du glucose peut comprendre un affichage pour afficher les résultats de la mesure.

WO2005001680 concerne une interface utilisateur pour un appareil de diagnostic médical portatif et le procédé d'utilisation associé. L'appareil de diagnostic médical comprend un écran tactile permettant d'afficher une interface utilisateur graphique (GUI) comprenant des sélecteurs virtuels. Un port est destiné à recevoir un échantillon placé sur une bandelette de test. Un détecteur, connecté avec un microprocesseur intégré dans l'appareil, permet l'analyse de l'échantillon.

US20100249965 concerne un dispositif pour la mesure du glucose destiné à recevoir une bandelette et à être connecté aux ports audio d'un dispositif portable.

WO03004160 concerne dispositif et un procédé destinés à la mise en oeuvre d'un dosage ou d'une réaction électrochimique faisant intervenir la conductivité et/ou le courant électrique de manière à réaliser une réduction, une oxydation ou une réaction de transfert ionique, des mesures de conductimétrie et/ou d'impédance, ou à créer un champ électrique dans une solution, ou à effectuer une combinaison de ceux-ci. Le dispositif comporte au moins une micro-puce présentant un micro-canal ou un réseau de micro-canaux pourvue d'une extrémité en pointe servant à prélever un échantillon fluidique et/ou à déposer un échantillon fluidique, d'une extrémité de connexion micro-fluidique et d'une électrode intégrée dans le micro-canal.

### Bref résumé de l'invention

Un but de la présente invention est de proposer un dispositif pour l'analyse d'un échantillon de fluide qui soit exempt des limitations des puces micro-fluidiques connues.

Un autre but de l'invention est de proposer un dispositif pour l'analyse d'un échantillon de fluide plus modulaire, flexible et moins cher que les solutions connues.

Selon l'invention, ces buts sont atteints notamment au moyen d'un dispositif micro-fluidique selon la revendication 1, d'un dispositif programmable avec un écran tactile capacitif selon la revendication 6, d'un usage d'un dispositif programmable avec un écran tactile capacitif selon la revendication 7, d'une méthode pour l'analyse d'un échantillon de fluide selon la revendication 8, d'un moyen de stockage non transitoire de données lisible par ordinateur selon la revendication 9 et d'un système selon la revendication 10.

Cette présente invention exploite les méthodes de détection capacitive d'un dispositif programmable avec un écran tactile capacitif comme interface entre l'échantillon de fluide et un outil d'analyse. L'idée proposée ici est d'utiliser dispositif programmable avec un écran tactile capacitif, par exemple une tablette tactile, comme interface et aussi comme unité de calcul pour l'analyse d'un dispositif micro-fluidique, par exemple une puce micro-fluidique.

L'écran tactile capacitif du dispositif programmable peut être assimilé à un périphérique fonctionnant en entrée/sortie qui pourrait être exploitée comme interface de détection. En effet les dispositifs programmables avec écrans tactiles capacitifs sont aujourd'hui produits en quantité industrielle et abordables pour un marché de grande consommation. De plus, ces dispositifs bénéficient d'une puissance de calcul accrue avec des micro-processeurs puissants et qui, grâce aux progrès de l'industrie microélectronique, ont une faible consommation.

Dans une variante le dispositif programmable avec un écran tactile capacitif peut être configuré pour permettre l'analyse d'un dispositif micro-fluidique de différents types ou de plusieurs dispositifs micro-fluidiques, du même type ou de différents types. Dans une autre variante plusieurs dispositifs programmables avec écran tactile capacitif peuvent être utilisés en parallèle et communiquer entre eux les résultats des analyses.

A la différence des solutions connues, qui nécessitent un capteur, un bus de communication avec une unité d'analyse et/ou d'affichage et une telle unité d'analyse et/ou d'affichage, la présente invention combine les trois fonctions (détection, analyse et affichage) en une seule grâce à l'utilisation d'un dispositif programmable avec un écran tactile capacitif.

De plus, l'utilisation d'un dispositif programmable avec un écran tactile capacitif déjà existant sur le marché permet de réduire non seulement les coûts de développement mais aussi de toucher un plus grand marché.

### Brève description des figures

Des exemples de mise en oeuvre de l'invention sont indiqués dans la description illustrée par les figures annexées dans lesquelles :
La figure 1 illustre une vue de section du dispositif programmable avec un écran tactile capacitif interfacé avec le dispositif micro-fluidique.
La figure 2 illustre un schéma électrique équivalent du dispositif programmable avec un écran tactile capacitif et du dispositif micro-fluidique.
La figure 3 illustre vue tridimensionnelle d'un mode de réalisation du dispositif programmable avec un écran tactile capacitif.
La figure 4 illustre un mode de réalisation du dispositif micro-fluidique selon l'invention.
La figure 5 illustre une vue de section d'une autre variante du dispositif programmable avec un écran tactile capacitif modifié interfacé avec le dispositif micro-fluidique.

### Exemple(s) de mode de réalisation de l'invention

La présente invention concerne un dispositif micro-fluidique 1 pour l'analyse d'un échantillon de fluide utilisant comme interface, moyen d'analyse de l'échantillon et d'affichage des résultats de l'analyse un dispositif programmable avec un écran tactile capacitif 2, c'est-à-dire basé sur une technologie dite capacitive. Le fluide de l'échantillon peut être un liquide, par exemple, mais de façon non limitative, du sang, de l'urine, du sérum, du plasma sanguin, de la salive, des sécrétions, ou un gaz, par exemple, mais de façon non limitative de l'haleine.

Les dispositifs programmables avec un écran tactile actuel sont basés principalement sur deux technologies : l'une résistive l'autre capacitive.

La technologie résistive consiste à superposer deux films conducteurs transparents séparés par un léger gap. Lors d'une action sur le dispositif, la pression exercée par le toucher crée une déformation mécanique du film supérieur et va rentrer en contact avec le film inférieur créant de ce fait un contact électrique entre ces deux films. La mesure de la résistance permet de détecter et localiser le contact. L'avantage de cette technologie est la possibilité d'utiliser aussi bien des objets que les doigts pour autant que la pression soit suffisante pour déformer suffisamment le film supérieur. En revanche, ces types des dispositifs auront tendance à s'user et à se rayer plus facilement en raison de la faible résistance mécanique du film supérieur.

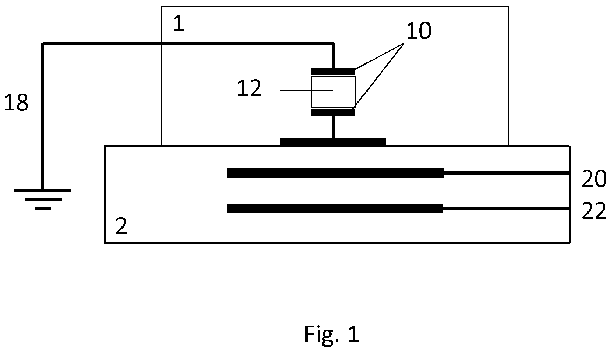

La technologie capacitive consiste à noyer deux réseaux d'électrodes transparentes composés en ITO (Indium Tin Oxyde), le premier réseau composé par d'électrodes de détection 20 et le deuxième par d'électrodes d'excitation 22, visibles en section sur la figure 1, les deux réseaux étant disposés perpendiculairement l'une par rapport à l'autre dans le dispositif tactile. Lors d'un toucher sur l'écran du dispositif avec un doigt ou un n'importe quel objet conducteur, le champ électrique généré par le réseau d'électrodes d'excitation 22 est perturbé et une partie des charges délivrées par le réseau d'électrodes d'excitation 22 traversent le doigt et vont à la terre. Le déficit de charges est alors détecté par le réseau d'électrodes de détection 20 qui repère la localisation du toucher par le biais d'un processeur.

L'avantage de cette technique par rapport à celle résistive est qu'il n'y pas de déformation mécanique de l'écran du dispositif, qui est donc plus solide dans le temps grâce à l'utilisation des verres résistant. Un autre avantage considérable est la possibilité de détecter plusieurs touchers simultanément (« multitouche »), ce qui n'est pas possible maintenant avec une technologie de type résistif.

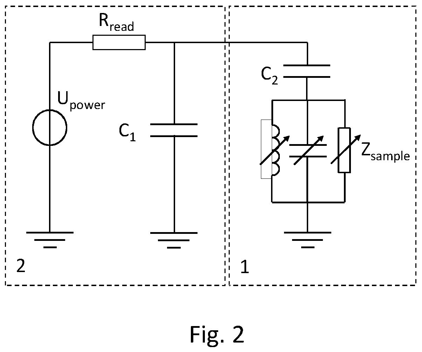

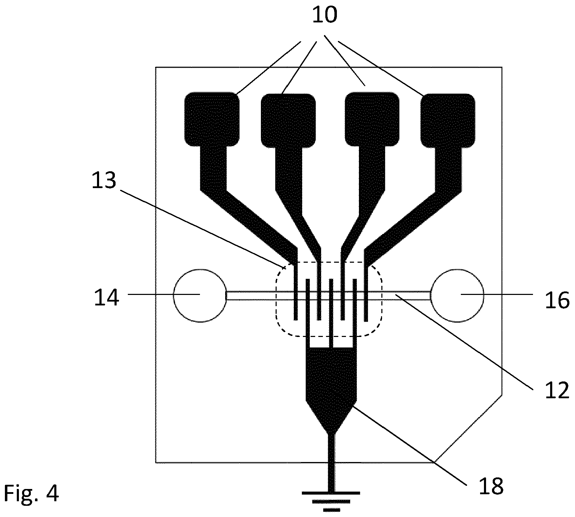

Selon l'invention, un dispositif avec un écran tactile à technologie capacitive 2 peut exploiter la variation d'impédance Zₛₐₘₚₗₑ, visible sur la figure 2, causée par un dispositif micro-fluidique 1. Dans ce cas, la variation d'impédance, par exemple une variation de capacité, ne sera pas générée par un toucher du doigt, mais par la variation de la composition ou des caractéristiques d'un échantillon placé sur une surface 121 entre deux électrodes 10 du dispositif micro-fluidique 1 (figures 1 et 4).

Avantageusement le dispositif programmable avec écran tactile capacitif 2 comprend un processeur exécutant un logiciel pour réaliser une détection du dispositif micro-fluidique 1. La détection permet, à l'aide d'un logiciel stocké dans le dispositif programmable 2 d'interpréter la quantité de charges en déficit et donc analyser l'échantillon de fluide. La quantité de charges perdues sera analysée en fonction de la position sur le dispositif tactile capacitif 2 et du temps. De plus, si le dispositif programmable avec écran tactile capacitif 2 est aussi « multitouche », il permet de gérer de façon indépendante plusieurs zones de lecture simultanément pour des analyses multiples.

Selon l'invention, un dispositif micro-fluidique 1 est agencé pour s'interfacer directement sur la surface d'affichage 24 d'un dispositif programmable avec écran tactile capacitif 2, par exemple une tablette tactile ou un téléphone multifonctions (« smartphone ») qui détectera la présence du dispositif micro-fluidique 1, analysera les informations provenant du dispositif micro-fluidique 1 en fonction de la composition de l'échantillon à analyser et affichera les résultats de cette analyse sur l'écran tactile capacitif.

Le dispositif programmable avec écran tactile capacitif 2 comprend avantageusement une mémoire exécutant un logiciel pour analyser les variations de charge provoquées par le dispositif micro-fluidique 1 sur l'écran tactile capacitif du dispositif programmable 2, et pour en déduire des résultats d'analyse de l'échantillon de fluide.

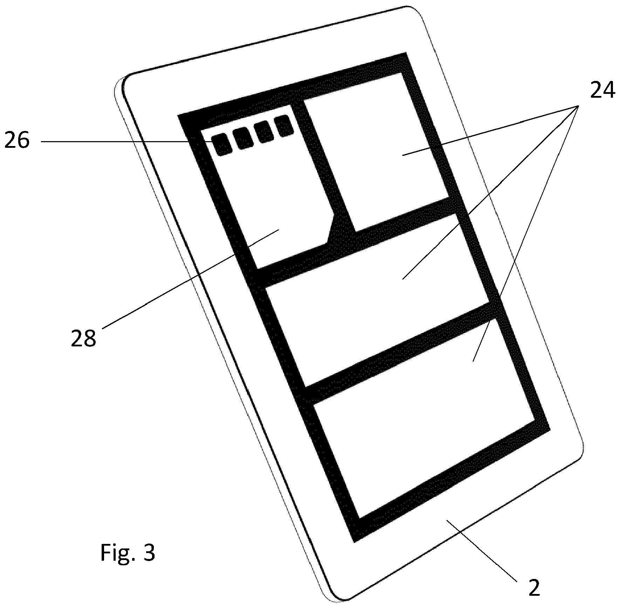

Selon l'invention l'échantillon serait tout d'abord injecté dans le dispositif micro-fluidique 1, qui est à usage unique, puis posé simplement sur une zone ou surface de lecture 28 définie sur la surface d'affichage 4 du dispositif programmable avec écran tactile capacitif 2, visible sur la figure 3. Cette surface 28 du dispositif programmable destinée à s'interfacer avec le dispositif micro-fluidique comprend une surface 26 destinée à s'interfacer avec au moins une électrode 10 du dispositif micro-fluidique 1. Les surfaces 26 et 28 seront affichées sur le dispositif programmable 2 avant l'appui du dispositif micro-fluidique 1. Le dispositif programmable avec écran tactile capacitif peut comprendre plusieurs surfaces 28.

Après avoir analysé les informations provenant du dispositif micro-fluidique 1 en fonction de la composition de l'échantillon à analyser, le dispositif programmable avec écran tactile capacitif 2 affiche les résultats de l'analyse.

Le dispositif micro-fluidique 1 est composé d'une ou plusieurs électrodes 10 pour s'interfacer avec le dispositif programmable avec écran tactile capacitif 2 et d'une ou plusieurs entrées/sorties 14 respectivement 16 d'au moins un micro-canal 12 pour l'introduction de l'échantillon à diagnostiquer (figure 4). La surface de détection 13 est localisée dans la partie du micro-canal 10 qui est superposée au réseau d'électrodes 10 en peigne. L'analyte à tester est placée sur une ou plusieurs surfaces 121 des canaux 12 en regard de la zone de détection 13.

Le dispositif micro-fluidique 1 est jetable et à usage unique. Dans une variante il est réalisé en polymère grâce à des méthodes de micro-fabrication, par exemple des méthodes de thermoformage, maitrisées et bien adaptées pour une production industrielle à faible coût.

Dans une autre variante, le dispositif micro-fluidique 1 peut être associé à une électronique externe d'amplification d'un signal ou pour actionner une plusieurs des ses parties, par exemple micro-valves, pompes.

Dans une variante, le dispositif micro-fluidique 1 est réalisé en plusieurs parties, dont l'une peut être à usage unique tandis que l'autre peut être à usage unique également, ou de préférence réutilisable. Dans un mode de réalisation, le dispositif micro-fluidique 1 comporte une portion jetable avec une ou plusieurs surfaces 121 pour l'échantillon à tester, ainsi que des électrodes, et un dispositif intermédiaire réutilisable et destiné à être placé sur l'écran tactile. La portion jetable vient enfichée et/ou reliée électriquement au dispositif intermédiaire, de manière à ce que les électrodes de la portion jetable soient en connexion électrique avec des électrodes du dispositif intermédiaire. Le dispositif intermédiaire n'est ainsi pas en contact avec le fluide à tester, et peut être réutilisé. Les électrodes du dispositif intermédiaire sont alors en contact électrique avec le circuit électrique de la portion jetable, dont l'impédance dépend du fluide à tester. Cette impédance variable est détectée par les électrodes capacitives de l'écran tactile 2.Le dispositif micro-fluidique 1 assure un contact électrique des électrodes 10 avec la masse 18 permettant de refermer le circuit de circulation des charges électriques.

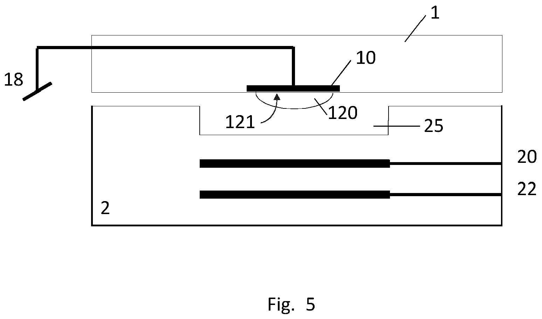

Dans une variante, le fluide se trouve directement en contact avec l'écran capacitif. Cela peut être réalisé de différentes manières :
- Dans la variante illustrée sur la figure 5, le canal, ou au moins un ou plusieurs logements 25, est fabriqué directement dans l'écran afin de réduire la distance électrodes/fluide et ainsi augmenter le signal et/ou diminuer le bruit. Le dispositif microfluidique referme le canal ou le logement pour s'interfacer avec l'écran. L'analyte peut être en contact avec le dispositif microfluidique ; ou avec l'écran tactile ; ou avec les deux surfaces simultanément.
- Dans une autre variante non représentée, un canal ouvert est fabriqué sous la surface inférieure du dispositif microfluidique qui est refermé en se plaquant avec la surface non modifiée de l'écran.
- Dans une troisième variante, le canal est fabriqué en partie dans l'écran et en partie dans le dispositif microfluidique.

Dans la variante de la figure 5, le dispositif microfluidique 1 coopère avec un dispositif programmable avec écran tactile capacitif modifié 2. Dans cet exemple, la surface du dispositif tactile comprend un ou plusieurs logements 25, par exemple des logements de forme rectangulaire, ronde ou sous forme de canaux en lignes ou en réseau par exemple. Ces logements sont directement gravés ou produits dans la surface extérieure 24 du verre recouvrant l'écran tactile, avec une profondeur par exemple inférieure à 2 millimètres. Les logements 25 permettent de loger l'analyte (goutte ou un filet de liquide ou gaz) à tester placé directement sur une surface 121 sous la surface inférieure du dispositif microfluidique 1, qui est plane dans cet exemple. Dans un autre mode de réalisation, le liquide à analyser est placé directement sur l'écran capacitif modifié 2, par exemple directement dans les logements 25, ou à l'extrémité d'un canal qui amène le fluide dans ces logements par capillarité. Le liquide peut aussi être introduit à l'extrémité d'un canal dans le dispositif microfluidique, et s'écouler par capillarité ou gravité jusque dans le logement 25 gravé dans l'écran du dispositif 25.

Dans une variante non illustrée, les logements sont constitués par des trous ou des trous borgnes dans une plaque plaquée sur la surface tactile du dispositif tactile 2, afin de créer une surépaisseur permettant de créer des logements suffisamment profond pour y loger des gouttes de liquide à tester au-dessus du dispositif tactile.

Il est aussi possible de créer sur cette plaque de surépaisseur, ou d'afficher sur l'écran tactile 2, des marques pour positionner correctement le dispositif microfluidique 1.

Ce dispositif est ainsi plus facile à utiliser pour certaines applications ; il suffit de déposer une goutte de liquide à un ou des endroits prédéfinis sous la surface inférieure du dispositif microfluidique 1, puis d'appliquer ce dispositif microfluidique au bon endroit contre l'écran du dispositif tactile 2 afin que les gouttes de liquide à tester se trouvent dans le ou les logements 25 prévus à cet effet durant le test. Il est aussi possible de prévoir des canaux à travers le dispositif microfluidique 1 afin d'amener par capillarité le liquide (ou le gaz par un flux entrant) introduit à un autre endroit dans les surfaces prédéfinies en face des logements 25.

Le dispositif microfluidique de la figure 5 peut aussi dans une variante non illustrée comporter une surface inférieure qui n'est pas plane, par exemple avec des logements en face des logements 25, afin de pouvoir recevoir des volumes d'analytes plus important sans devoir graver des logements trop importants dans le dispositif 2.

La figure 2 illustre un schéma électrique équivalent de l'interface entre le dispositif programmable avec écran tactile capacitif 2 et le dispositif micro-fluidique 1 : le dispositif programmable 2 est schématisé avec un générateur de tension U_{power}, une capacité C₁ et une résistance R_{read}. L'accouplement entre le dispositif programmable avec écran tactile capacitif 2 et le dispositif micro-fluidique 1 est schématisé avec une capacité C₂. Le dispositif micro-fluidique 1 est schématisé avec une impédance Zₛₐₘₚₗₑ, variable en fonction de l'échantillon à analyser, et connectée à terre.

Un logiciel stocké dans une mémoriel du dispositif programmable avec écran tactile capacitif 2 permet de lire et analyser directement les données lues sur le dispositif micro-fluidique 1 puis d'afficher les résultats sur l'écran tactile capacitif du dispositif 2. Le logiciel est donc capable de quantifier les variations de l'impédance du dispositif programmable 2 crées par le dispositif micro-fluidique 1 et d'en déterminer une ou plusieurs caractéristiques de l'échantillon à analyser.

Dans une variante le logiciel peut analyser plusieurs dispositifs micro-fluidiques 1 simultanément pour autant que la surface de l'écran tactile capacitif du dispositif programmable 2 et la capacité de calcul de son processeur le permettent.

Dans une variante, le logiciel permet de commander le contrôleur d'écran tactile, afin d'éviter ou de modifier le filtrage habituellement effectué par ce contrôleur pour écarter les variations d'impédance qui ne sont pas produites par un doigt de l'utilisateur. Dans une variante, un nouveau firmware est chargé dans ce contrôleur. Dans une variante, le logiciel permet d'éviter ou de modifier le traitement logiciel des touches habituellement effectué, en plus du traitement par le contrôleur d'écran tactile, par le système d'exploitation du dispositif 2.

Le contrôleur pilotant l'écran tactile capacitif pourra fonctionner en mode hybride : un mode « standard » et un mode « analyse échantillon(s) ». Dans le mode de fonctionnement « standard », le contrôleur fonctionne comme dans un dispositif tactile conventionnel, par exemple une tablette tactile ou un smartphone. Il est capable de distinguer les touchers volontaires (un ou plusieurs doigts) des touchers involontaires (paumes, oreilles, joues, etc.). Dans le mode « analyse échantillon(s) », le contrôleur est capable de détecter les variations de charges de l'écran interfacé avec le dispositif microfluidique dans une ou plusieurs zones spécifiques de détection de l'écran.

Dans un mode de réalisation, un contrôleur supplémentaire destiné au mode « analyse échantillon(s) » uniquement peut être mis en oeuvre en à un contrôleur conventionnel fonctionnant en mode « standard » pour traiter les signaux. Le basculement d'un mode à un autre peut se faire soit de façon automatique ou manuel via un logiciel d'interface exécuté par le dispositif 2.

Les surfaces 26 de lecture des électrodes et du dispositif micro-fluidique 1, ainsi que leurs nombres, peuvent être configurées en fonction du dispositif micro-fluidique 1 utilisé.

Le logiciel offre également la possibilité d'exporter les données des résultats directement à travers le dispositif programmable avec écran tactile capacitif 2. A cet effet le dispositif programmable 2 comprend des moyens sans et/ou avec fils pour exporter les résultats de l'analyse à un ordinateur externe, par exemple en utilisant une interface 3G/4G, Wifi, Bluetooth, USB, etc.

Les données exportées par internet ou d'autre modes de communication pourront être réinterprétées et stockées par du personnel compétent. Les données pourront être répertoriées sur un compte d'un utilisateur pour retracer un historique des analyses.

Dans une variante le dispositif programmable avec écran tactile capacitif 2 comprend aussi des moyens pour stoker les résultats de l'analyse.

Dans une autre variante les résultats de l'analyse ne seront pas affichés sur l'écran tactile capacitif du dispositif programmable 2, mais seront envoyés à un dispositif externe sur lequel ils seront affichés.

Puisque le dispositif micro-fluidique 1 n'intègre pas ou peu d'électronique de détection, le prix de revient est réduit comparé à d'autres systèmes intégrés. La partie jetable pourra être découplée de l'éventuelle partie électronique externe afin de réduire les coûts de fabrication de cette première.

L'analyse se faisant sur un dispositif programmable avec écran tactile capacitif existant, par exemple une tablette tactile ou des smartphones, aucun système d'analyse dédié ne doit être développé en conséquence. De plus, le fait d'utiliser un dispositif programmable avec écran tactile capacitif 2 pour s'interfacer avec un dispositif micro-fluidique 1 est une manière simple de diagnostic pouvant attirer des marché plus larges que des dispositifs plus robuste mais également plus couteux.

Le logiciel d'acquisition, d'analyse et d'affichage des données des résultats de l'analyse pourra être commercialisé via des plateformes de vente en ligne. Plusieurs logiciels pourront être commercialisés en fonction des besoins des différents utilisateurs (particuliers, personnel médical, médecins, organisations humanitaire).

Le dispositif micro-fluidique 1 dans une variante comprend une combinaison de nombre et/ou de forme d'électrodes qui permet au dispositif programmable avec écran tactile capacitif 1 de l'identifier et/ou d'identifier le(s) type(s) d'analyse à faire et/ou d'identifier l'utilisateur du dispositif micro-fluidique 1. Dans une variante préférentielle les électrodes ont une forme de code barre mono ou bidimensionnel. Dans une autre variante le dispositif micro-fluidique 1 comprend un tag RFID qui pourra être lu par un lecteur RFID intégré dans le dispositif programmable 2.

Dans une autre variante le dispositif micro-fluidique 1 comprend un revêtement pour favoriser l'interaction et l'analyse avec l'échantillon de fluide. Par exemple des enzymes ou des molécules pourrait être fixés préalablement sur l'électrode(s) 10 et/ou le micro canal (micro canaux) 12. De manière plus générale, la surface 121 pour placer l'analyte, par exemple l'électrode sur laquelle elle est placée, ou une autre portion des micro-canaux 12, peut subir une opération de fonctionnalisation chimique. Le but de la fonctionnalisation chimique de la surface est de reconnaitre uniquement des molécules spécifiques (contenues dans l'échantillon à analyser) qui vont adhérer à la surface. La variation de capacitance est alors due à la reconnaissance puis l'adhésion de cette molécule spécifique (et non les autres se trouvant dans l'échantillon).

Différentes surfaces 121 dans un même dispositif micro-fluidique 1 peuvent avoir différentes fonctionnalisation pour réagir chacune spécifiquement à un type précis de molécule pour différentes analyses.

Dans une autre variante les résultats de l'analyse sur l'échantillon de fluide peuvent être combinés à des données du dispositif programmable 2, qui pourrait comprendre par exemple un module GPS, pour faire des analyses statistiques et/ou suivre l'évolution géographique de ces résultats.

Des exemples non exhaustifs et non limitatifs d'applications de la présente invention sont
- Mesure du taux de glucose dans le sang pour les diabétiques.
- Mesure de la pollution de l'eau ou de la contamination alimentaire (virus, bactéries).
- Ethylotest pour la mesure du taux d'alcoolémie par le sang ou par l'haleine.
- Détection de substances explosives
- Identification d'allergies à partir d'un échantillon de sang.

### Numéros de référence employés sur les figures

- 1: Dispositif micro-fluidique
- 10: Electrode
- 12: Micro-canal
- 120: Volume d'analyte à tester
- 121: Surface pour placer l'échantillon à tester
- 13: Surface de détection
- 14: Entrée du micro-canal
- 16: Sortie du micro-canal
- 18: Contact électrique avec la masse
- 2: Dispositif programmable avec écran tactile capacitif
- 20: Electrode de détection
- 22: Electrode d'excitation
- 24: Surface d'affichage du dispositif tactile capacitif
- 25: Logement ou canal dans la surface 24
- 26: Surface du dispositif tactile capacitif destinée à s'interfacer avec au moins une électrode du dispositif micro-fluidique
- 28: Surface du dispositif tactile capacitif destinée à s'interfacer avec le dispositif micro-fluidique
- U_{power}: Alimentation du dispositif tactile capacitif
- C₁: Capacité du dispositif tactile capacitif
- R_{read}: Résistance du dispositif tactile capacitif
- C₂: Capacité d'accouplement entre le dispositif tactile capacitif et le dispositif micro-fluidique
- Zₛₐₘₚₗₑ: Impédance de l'échantillon de fluide

## Revendications

1. Dispositif micro-fluidique (1) pour l'analyse d'un échantillon de fluide comprenant
- au moins une première électrode (10) reliée à la masse (18),
- au moins une deuxième électrode (10) destinée à s'appuyer sur une surface (26) d'un dispositif programmable à écran tactile capacitif (2) lorsque ledit dispositif micro-fluidique (1) est placé sur ledit écran tactile,
- un micro-canal (12) comprenant une entrée (14) pour l'introduction dudit échantillon,
**caractérisé en ce que**
lesdites premières et deuxièmes électrodes comprennent des extrémités disposées en peigne,
- une partie dudit micro-canal (12) est superposée auxdites extrémités en peigne desdites électrodes,
lesdites premières et deuxièmes électrodes (10) sont agencées de manière à produire une variation mesurable de capacité sur l'écran tactile dudit dispositif programmable à écran tactile capacitif (2) lorsque ladite deuxième électrode appuie sur ladite surface (26) du dispositif programmable à écran tactile capacitif (2) lorsque ledit dispositif micro-fluidique (1) est placé sur ledit écran tactile capacitif, de manière à permettre l'analyse dudit échantillon de fluide.

2. Le dispositif selon la revendication 1, réalisé en polymère grâce à une méthode de micro-fabrication.

3. Le dispositif selon l'une des revendications 1 ou 2, comprenant au moins une micro-valve et/ou au moins une pompe.

4. Le dispositif selon la revendication 3, agencé pour être couplé avec une électronique externe d'amplification et/ou d'actionnement de ladite au moins une micro-valve et/ou de ladite au moins une pompe.

5. Le dispositif selon l'une des revendications 2 à 4, comprenant une surface de détection (13) entre ladite entrée (14) et une sortie (16) dudit micro-canal (12).

6. Le dispositif selon l'une des revendications 2 à 5, comprenant au moins une dite première ou deuxième électrode (10) sur la face dudit micro-canal en regard dudit dispositif programmable (2), et au moins une dite deuxième ou première électrode (10) sur la face opposée dudit micro-canal (12).

7. Un moyen de stockage non transitoire de données lisible par ordinateur, comprenant des instructions exécutées par une unité de contrôle informatisée pour l'analyse d'un échantillon de fluide introduit dans une entrée (14) d'au moins un micro-canal (10) d'un dispositif micro-fluidique (1) comprenant au moins une première électrode (10) reliée à la masse (18), au moins une deuxième électrode (10) destinée à s'appuyer sur une surface (26) d'un dispositif programmable à écran tactile capacitif (2) lorsque ledit dispositif micro-fluidique (1) est placé sur ledit écran tactile et un micro-canal (12) comprenant une entrée (14) pour l'introduction dudit échantillon, lesdites premières et deuxièmes électrodes comprennent des extrémités disposées en peigne, une partie dudit micro-canal (12) étant superposée auxdites extrémités en peigne desdites électrodes, lesdites instructions faisant exécuter à ladite unité de contrôle une méthode comprenant les étapes suivantes
- analyser les variations de charges provoquées par ledit dispositif micro-fluidique (1) sur ledit écran tactile capacitif et en déduire des résultats d'analyse dudit échantillon
- afficher sur ledit écran tactile capacitif (2) des résultats de l'analyse.

8. Méthode pour l'analyse d'un échantillon de fluide comprenant les étapes suivantes
- introduction dudit échantillon dans une entrée (14) d'un micro-canal d'un dispositif micro-fluidique (1), ledit dispositif micro-fluidique comprenant au moins une première électrode (10) reliée à la masse (18) et au moins une deuxième électrode (10), lesdites premières et deuxièmes électrodes comprenant des extrémités disposées en peigne, une partie dudit micro-canal (12) étant superposée auxdites extrémités en peigne desdites électrodes ;
- placer ledit dispositif micro-fluidique (1) sur l'écran tactile d'un dispositif programmable avec un écran tactile capacitif (2), ledit écran capacitif (2) comprenant une surface (26) destinée à s'interfacer avec ladite deuxième électrode (10), de façon à ce que ladite deuxième électrode (10) du dispositif micro-fluidique (1) appuie sur ladite surface (26, 28),
- analyser les variations de charge provoquées par ledit dispositif micro-fluidique (1) sur ledit écran tactile capacitif, et pour en déduire des résultats d'analyse dudit échantillon
- afficher sur ledit écran tactile capacitif des résultats de l'analyse.

9. Dispositif programmable avec un écran tactile capacitif (2) comprenant
- au moins une surface d'affichage (24) de l'écran tactile capacitif (2) pour afficher des résultats d'analyse;
- au moins une portion (28) de ladite surface d'affichage (24) étant destinée à s'interfacer avec le dispositif de l'une des revendications 1 à 6,
- au moins un processeur,
- le moyen de stockage non transitoire de données lisible par ordinateur selon la revendication 7.

10. Système comportant
- un dispositif programmable avec un écran tactile capacitif (2) comprenant
- au moins une surface d'affichage (24) pour afficher des résultats d'analyse ;
- au moins une portion (28) de ladite surface d'affichage (24), destinée à s'interfacer avec un dispositif micro-fluidique,
- au moins un processeur,
- le moyen de stockage non transitoire de données lisible par ordinateur selon la revendication 7,
- ledit dispositif micro-fluidique (1) selon l'une des revendications 1 à 6.

## Patentansprüche

1. Mikrofluidische Vorrichtung (1) zur Analyse einer Flüssigkeitsprobe, umfassend:
- mindestens eine erste Elektrode (10), welche an der Masse (18) angeschlossen ist;
- mindestens eine zweite Elektrode (10), welche zur Auflage auf eine Fläche (26) einer programmierbaren Vorrichtung mit kapazitivem Tastbildschirm (2) bestimmt ist, wenn die besagte mikrofluidische Vorrichtung (1) auf dem besagten Tastbildschirm angeordnet wird,
- einen Mikrokanal (12), der einen Einlass (14) zum Einführen der besagten Probe aufweist,
**dadurch gekennzeichnet, dass**
die besagten ersten und zweiten Elektroden gekämmte Enden aufweisen,
- ein Teil des besagten Mikrokanals (12) auf besagten gekämmten Enden von besagten Elektroden überlagert ist,
- die besagten ersten und zweiten Elektrode (10) so angeordnet sind, um eine messbare Kapazitätsänderung auf dem Tastbildschirm der besagten programmierbaren Vorrichtung mit kapazitivem Tastbildschirm (2) zu erzeugen, wenn die besagte zweite Elektrode auf die besagte Fläche (26) der besagten programmierbaren Vorrichtung mit kapazitivem Tastbildschirm (2) aufliegt, wenn die besagte mikrofluidische Vorrichtung (1) auf dem besagten Tastbildschirm angeordnet wird, um die Analyse der besagten Fluidprobe zu ermöglichen.

2. Vorrichtung gemäss Anspruch 1, hergestellt aus einem Polymer unter Verwendung eines Mikroherstellungsverfahrens.

3. Vorrichtung gemäss einem der Ansprüche 1 oder 2, umfassend mindestens ein Mikroventil und/oder mindestens eine Pumpe.

4. Vorrichtung gemäss Anspruch 3, welch dazu eingerichtet ist, um mit einer externen Verstärkungs- und/oder Betätigungselektronik des besagten mindestens einen Mikroventils und/oder der besagten mindestens einen Pumpe gekoppelt zu werden.

5. Vorrichtung gemäss einem der Ansprüche 2 bis 4, umfassend eine Erfassungsfläche (13) zwischen dem besagten Einlass (14) und einem Auslass (16) des besagten Mikrokanals (12).

6. Vorrichtung gemäss einem der Ansprüche 2 bis 5, umfassend mindestens eine besagte erste oder zweite Elektrode (10) auf der der besagten programmierbaren Vorrichtung (2) gegenüberliegenden Fläche des besagten Mikrokanals, und mindestens eine besagte zweite oder erste Elektrode (10) auf der anderen Fläche des besagten Mikrokanals (12).

7. Computerlesbare nichtflüchtige Datenspeichereinrichtung mit Anweisungen, die von einer computergesteuerten Steuereinheit zum Analysieren einer Flüssigkeitsprobe ausgeführt werden, welche in einen Einlass (14) mindestens eines Mikrokanals (10) einer mikrofluidischen Vorrichtung (1) eingeführt wird, welche umfasst: mindestens eine erste Elektrode (10), welche an der Masse (18) angeschlossen ist, mindestens eine zweite Elektrode (10), welche zur Auflage auf eine Fläche (26) einer programmierbaren Vorrichtung mit kapazitivem Tastbildschirm (2) bestimmt ist, wenn die besagte mikrofluidische Vorrichtung (1) auf dem besagten Tastbildschirm angeordnet wird; und einen Mikrokanal (12), der einen Einlass (14) zum Einführen der besagten Probe aufweist, wobei die besagten ersten und zweiten Elektroden kammförmig angeordnete Enden aufweisen, wobei ein Teil des besagten Mikrokanals (12) auf die besagten kammförmigen Enden der besagten Elektroden überlagert ist, wobei die besagten Anweisungen die besagte Steuereinheit zur Ausführung eines Verfahrens mit den folgenden Schritten veranlassen:
- Analysieren der durch die besagte mikrofluidische Vorrichtung (1) auf dem besagten kapazitiven Tastbildschirm verursachten Ladungsänderungen und Ableitung davon der Analyseergebnisse der besagten Probe;
- Anzeigen auf dem besagten kapazitiven Tastbildschirm (2) der Ergebnisse der Analyse.

8. Verfahren zum Analysieren einer Fluidprobe, mit den folgenden Schritten:
- Einführen der besagten Probe in einen Einlass (14) eines Mikrokanals einer mikrofluidischen Vorrichtung (1), wobei die besagte mikrofluidische Vorrichtung umfasst: mindestens eine erste Elektrode (10), welche an der Masse (18) angeschlossen ist, und mindestens eine zweite Elektrode (10), wobei die besagten ersten und zweiten Elektroden kammförmig angeordnete Enden aufweisen, wobei ein Teil des besagten Mikrokanals (12) auf die besagten kammförmigen Enden der besagten Elektroden überlagert ist;
- Platzieren der besagten mikrofluidischen Vorrichtung (1) auf dem Tastbildschirm einer programmierbaren Vorrichtung mit einem kapazitiven Tastbildschirm (2), wobei der besagte kapazitive Bildschirm (2) eine Fläche (26) umfasst, welche dazu geeignet ist, mit der besagten zweiten Elektrode (10) eine Schnittstelle zu erstellen, so dass die besagte zweite Elektrode (10) der mikrofluidischen Vorrichtung (1) auf die besagte Fläche (26, 28) aufliegt,
-- Analysieren der durch die besagte mikrofluidische Vorrichtung (1) auf dem besagten kapazitiven Tastbildschirm verursachten Ladungsänderungen und Ableitung davon der Analyseergebnisse der besagten Probe;
- Anzeigen auf dem besagten kapazitiven Tastbildschirm (2) der Ergebnisse der Analyse.

9. Programmierbare Vorrichtung mit kapazitivem Tastbildschirm (2), umfassend:
- mindestens eine Anzeigefläche (24) des kapazitiven Tastbildschirms (2) zum Anzeigen der Analyseergebnisse;
- wobei mindestens ein Teil (28) der besagten Anzeigefläche (24) dazu bestimmt ist, mit der Vorrichtung von einem der Ansprüche 1 bis 6 eine Schnittstelle zu erstellen,
- mindestens einen Prozessor,
- die computerlesbare nichtflüchtige Datenspeichereinrichtung gemäss Anspruch 7.

10. System, umfassend
- eine programmierbare Vorrichtung mit einem kapazitiven Tastbildschirm (2), umfassend:
- mindestens eine Anzeigefläche (24) zum Anzeigen der Analyseergebnisse;
- wobei mindestens ein Teil (28) der besagten Anzeigefläche (24) dazu bestimmt ist, mit einer mikrofluidischen Vorrichtung eine Schnittstelle zu erstellen,
- mindestens einen Prozessor,
- die computerlesbare nichtflüchtige Datenspeichereinrichtung gemäss Anspruch 7,
- die besagte mikrofluidische Vorrichtung (1) gemäss einem der Ansprüche 1 bis 6.

## Claims

1. Microfluidic device (1) for the analysis of a fluid sample comprising
- at least one first electrode (10) connected to ground (18),
- at least one second electrode (10) intended to lean on a surface (26) of a capacitive touch screen programmable device (2) when said microfluidic device (1) is placed on said touch screen,
- a micro-channel (12) comprising an inlet (14) for introducing said sample,
**characterized in that**
said first and second electrodes comprise comb-shaped ends,
a part of said micro-channel (12) is superimposed on said comb-shaped ends of said electrodes,
said first and second electrodes (10) are arranged to produce a measurable capacitance variation on the touch screen of said capacitive touch screen programmable device (2) when said second electrode lays on said surface (26) of the capacitive touch screen programmable device (2) when said microfluidic device (1) is placed on said capacitive touch screen, so as to allow the analysis of said fluid sample.

2. The device according to claim 1, made of polymer using a micro-manufacturing method.

3. The device according to one of claims 1 or 2, comprising at least one micro-valve and/or at least one pump.

4. The device according to claim 3, arranged to be coupled with an external electronic of amplification and/or of actuation of said at least one micro-valve and/or said at least one pump.

5. The device according to one of claims 2 to 4, comprising a detection surface (13) between said inlet (14) and an outlet (16) of said micro-channel (12).

6. The device according to one of claims 2 to 5, comprising at least one said first or second electrode (10) on the face of said micro-channel facing said programmable device (2), and at least one said second or first electrode (10) on the opposite side of said micro-channel (12).

7. A computer-readable, non-transitory data storage means comprising instructions executed by a computerized control unit for analyzing a sample of fluid introduced into an inlet (14) of at least one micro-channel (10) of a microfluidic device (1) comprising at least a first electrode (10) connected to the ground (18), at least a second electrode (10) intended to lean on a surface (26) of a capacitive touch screen programmable device (2) when said microfluidic device (1) is placed on said touch screen and a micro-channel (12) comprising an input (14) for introducing said sample, said first and second electrodes comprise comb-shaped ends, a portion of said micro-channel (12) being superimposed on said comb-shaped ends of said electrodes, said instructions causing said control unit to perform a method comprising the following steps:
- analyzing the variations of charges caused by said microfluidic device (1) on said capacitive touch screen and deducing from it analysis results of said sample,
- displaying on said capacitive touch screen (2) results of the analysis.

8. Method for analyzing a fluid sample comprising the following steps:
- introducing said sample into an inlet (14) of a micro-channel of a microfluidic device (1), said microfluidic device comprising at least a first electrode (10) connected to the ground (18) and to the at least one second electrode (10), said first and second electrodes comprising combed ends, a portion of said microchannel (12) being superimposed on said comb-shaped ends of said electrodes;
- placing said micro-fluidic device (1) on the touch screen of a programmable device with a capacitive touch screen (2), said capacitive screen (2) comprising a surface (26) intended to interface with said second electrode (10), so that said second electrode (10) of the microfluidic device (1) lays on said surface (26, 28),
- analyzing the load variations caused by said microfluidic device (1) on said capacitive touch screen, and to deduce from it analysis results from said sample,
- displaying on said capacitive touch screen results of the analysis.

9. Programmable device with a capacitive touch screen (2) comprising:
- at least one display surface (24) of the capacitive touch screen (2) for displaying analysis results;
- at least a portion (28) of said display surface (24) being intended to interface with the device of one of claims 1 to 6,
- at least one processor,
- the computer-readable non-transitory data storage means according to claim 7.

10. System comprising
- a programmable device with a capacitive touch screen (2) comprising
- at least one display surface (24) for displaying analysis results;
- at least a portion (28) of said display surface (24) intended to interface with a microfluidic device,
- at least one processor,
- the computer-readable non-transitory data storage means according to claim 7,
- said micro-fluidic device (1) according to one of claims 1 to 6.
